# DEMANDE DE BREVET EUROPEEN

(11) **EP 2 228 381 A2**
(43) Date de publication de la demande: **15.09.2010**
(21) Numéro de dépôt: 10157084.4
(22) Date de dépôt: 10.05.2005
(51) Int. Cl.: C07K 5/06, A61K 38/05, A23L 1/305

(54) **Conjugués dipeptidiques antagonistes de l'alpha-MSH**

(30) Priorité: 11.05.2004 FR 0405069; 22.10.2004 FR 0411279
(62) Demande divisionnaire de: 05770970.1
(71) Demandeur: Centre National de la Recherche Scientifique (CNRS), 75016 Paris (FR); INSTITUT EUROPEEN DE BIOLOGIE CELLULAIRE, 31520 Ramonville St Agne (FR); Université de Montpellier I, 34000 Montpellier (FR); Universite Montpellier 2 Sciences Et Techniques, 34000 Montpellier (FR)
(72) Inventeur: Pinel, Anne-Marie, 31400 Toulouse (FR); Verdie, Pascal, 34270 Saint Mathieu de Tréviers (FR); Dubs, Pascaline, 92100 Boulogne Billancourt (FR); Martinez, Jean, 34720 Caux (FR); Subra, Gilles, 34980 Saint Gely du Fesc (FR)
(74) Mandataire: Warcoin, Jacques

(57) **Abrégé**

La présente invention concerne un conjugué dipeptidique de formule générale 1 suivante AA2-AA1-NH₂ dans laquelle A représente le radical correspondant à un acide monocarboxylique de formule générale II suivante HOOC-R dans laquelle R représente un radical aliphatique en C₁-C₂₄ linéaire ou ramifié, éventuellement substitué par un groupe hydroxyle, pouvant comporter une ou plusieurs insaturations, avantageusement de 1 à 6 insaturations, et/ou pouvant comporter un groupe phényle ou l'acide lipoïque ou sa forme réduite l'acide dihydrolipoïque ou la N-lipoyllysine, AA1 et AA2 représentent des acides aminés identiques ou différents choisis dans le groupe constitué par Ala, Asn, Cys, Gln, Gly, Ile, Leu, Met, Phe, Pro, Ser, Thr, Trp, Tyr, Val, Asp, Glu, Arg, His, Lys, Orn, Dap, Dab les homoacides aminés correspondants et les béta-acides aminés correspondants sous forme d'énantiomères ou de diastéréoisomères ainsi que leurs mélanges, y compris les mélanges racémiques.

## Description

La présente invention concerne de nouveaux conjugués dipeptidiques antagonistes de l'alpha-MSH et leur utilisation en tant que médicament ou en tant que dépigmentant.

Les récepteurs de la mélanocortine appartiennent à la superfamille de sept récepteurs transmembranaires couplés à la protéine G et ils stimulent la voie de la transduction du signal de l'AMPc (Cone et al. Recent Prog. Horm. Res. 1996, 51, pages 287-317). Le système de la mélanocortine est impliqué dans de multiples voies physiologiques y compris la pigmentation, l'inflammation, la fonction érectile, le comportement alimentaire, l'homéostasie énergétique, l'homéostasie pondérale et la fonction des glandes exocrines. Les ligands des agonistes endogènes pour ces récepteurs de la mélanocortine sont dérivés par modification post-traductionnelle du transcrit du gène de la proopiomélanocortine, qui lors du traitement différentiel entraîne la génération des hormones α, β et y stimulant les mélanocytes (MSH) et de la corticotrophine (ACTH). Les sous-types des récepteurs de la mélanocortine sont activés par tous les peptides de la mélanocortine endogène, à l'exception du récepteur de la mélanocortine MC₂ qui est uniquement stimulé par la corticotrophine. La famille des récepteurs de la mélanocortine a aussi deux antagonistes endogènes, l'agouti et la protéine apparentée à l'agouti (AGRP) (Lu et al. Nature 1994, 371, pages 799-802, Ollmann et al., Science 1997, pages 135-138, Shulter et al., Genes Dev. 1997, 11, pages 593-602) qui sont les seuls antagonistes connus existant à l'état naturel de ces récepteurs couplés à la protéine G découverts à ce jour. Ce sont des polypeptides de, respectivement, 132 et 49 résidus d'acides aminés. Les ligands des récepteurs de la mélanocortine les mieux étudiés sont ceux des récepteurs MC₁ de la mélanocortine de la peau qui sont impliqués dans la pigmentation et la coloration du pelage des animaux (Hruby et al. Ann. N.Y. Acad. Sci. 1993, 680, pages 51-63 ; Lerner et al. Nature 1961, pages 189, 176 ; Mountjoy, et al. Science 1992, 257, pages 1248-1251).
Le nonapeptide *153 N-6* (Jayawickreme et al., J. Biol. Chem. 1994, 269, pages 29846-29854) (H-Met-Pro-D-Phe-Arg-d-Trp-Phe-Lys-Pro-Val-NH₂: Ki = 11 nM) est un antagoniste de synthèse du récepteur MC₁. Toutefois, ce composé est de haut poids moléculaire et possède donc une activité thérapeutique ou cosmétique très limitée. En effet, sa taille le rend difficile à optimiser et sa biodisponibilité est limitée. De plus, il est cher et difficile à préparer.
Le tripeptide D-Trp-Arg-Leu-NH₂ (Proc. Natl. Acad. Sci. (1995), 92, pages 2894 - 2898) a également une activité antagoniste. Toutefois, il contient le tryptophan qui est un acide aminé instable et peut donc poser des problèmes de stabilité au stockage.

EP 1 174 437 décrit des di ou tripeptides comportant un groupe naphtyle et en particulier un groupe naphtylalanyle. Toutefois, la présence du groupe naphtyle augmente le prix de fabrication du produit. Par ailleurs, dans certains pays comme le Japon, les peptides à base d'acide aminé non naturel ne peuvent être vendus pour des applications cosmétiques. En outre aucune activité de dipeptides n'est indiquée.

Les inventeurs ont découvert de façon surprenante que des dipeptides conjugués au niveau du C-terminal avec des acides carboxyliques ont une activité antagoniste de l'alpha MSH. Ces antagonistes ont un très faible poids moléculaire, sont donc faciles à optimiser, ont une bonne biodisponibilité et sont très faciles à préparer.

La présente invention concerne donc un conjugué dipeptidique de formule générale I suivante :

A-AA2-AA1-NH₂ I

dans laquelle
A représente le radical correspondant à un acide monocarboxylique de formule générale II suivante :

HOOC-R II

dans laquelle R représente
- un radical aliphatique en C₁-C₂₄ linéaire ou ramifié, éventuellement substitué par un groupe hydroxyle, pouvant comporter une ou plusieurs insaturations, avantageusement de 1 à 6 insaturations, et/ou pouvant comporter un groupe phényle
- ou l'acide lipoïque ou sa forme réduite l'acide dihydrolipoïque ou la N-lipoyl-lysine,
   AA1 et AA2 représentent des acides aminés identiques ou différents choisis dans le groupe constitué par Ala, Asn, Cys, Gln, Gly, Ile, Leu, Met, Phe, Pro, Ser, Thr, Trp, Tyr, Val, Asp, Glu, Arg, His, Lys, Orn, Dap, Dab, les homoacides aminés correspondants et les béta-acides aminés correspondants,
sous forme d'énantiomères ou de diastéréoisomères ainsi que leurs mélanges, y compris les mélanges racémiques.

Les acides aminés dans le conjugué dipeptidique de formule (I) peuvent avoir une configuration D, L ou DL si cela n'est pas spécifié autrement.
Ainsi, les conjugués dipeptidiques de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques font partie de l'invention.

Dans le cadre de la présente invention, on entend par :
- Ala, l'Alanine,
- Asn, l'Asparagine,
- Cys, la Cystéine,
- Gln, la Glutamine,
- Gly, la Glycine,
- Ile, l'Isoleucine,
- Leu, la Leucine,
- Met, la Méthionine,
- Phe, la Phénylalanine, ou ses analogues, en particulier un dérivé halogéné, en particulier la para-fluoro-Phe, la Homo-Phe, la para-nitro-Phe ou la phénylglycine,
- Pro, la Proline,
- Ser, la Sérine,
- Thr, la Thréonine,
- Trp, le Tryptophane,
- Tyr, la Tyrosine,
- Val, la Valine,
- Asp, l'Acide Aspartique,
- Glu, l'Acide Glutamique,
- Arg, l'Arginine,
- His, l'Histidine,
- Lys, la Lysine,
- Orn, l'Ornithine,
- Dap, l'Acide diaminopropionique,
- Dab, l'Acide diaminobutyrique.

Il est également précisé que les conjugués dipeptidiques mentionnés ci-dessus et faisant l'objet de la présente invention, sont obtenus sous la forme terminale NH₂ (autrement dit présentant une fonction amide).

Les dipeptides conjugués selon la présente invention sont liés sous forme de sels ou d'esters à l'acide de formule II. Les conjugaisons selon la présente invention peuvent être effectués en faisant réagir la fonction acide de l'acide aminé avec la fonction acide de l'acide de formule II, ou même il est possible de mettre à profit la présence d'une fonction hydroxy sur l'acide de formule II.
La présente invention concerne l'ensemble de ces conjugaisons ainsi que les conjugués non fonctionnels.
Les conjugaisons sont physiques ou chimique.

Avantageusement au moins un des acides aminés AA2 ou AA1, avantageusement les deux, représente un acide aminé basique, avantageusement choisi dans le groupe constitué par Arg, His, Dap, Dab, Orn ou Lys, avantageusement il s'agit de Arg.
De façon avantageuse AA2 représente un acide aminé basique avantageusement choisi dans le groupe constitué par Arg, His, Lys, Orn, Dap ou Dab, avantageusement il s'agit de Arg.
Avantageusement AA1 et/ou AA2 ne représentent pas Trp.
Avantageusement AA1 et/ou AA2 ne représentent pas Cys.
Avantageusement au moins un des acides aminés AA1 ou AA2 est choisi dans le groupe constitué par Ser et Pro. Avantageusement AA1 représente Pro.
Avantageusement AA2 représente Ser.
Avantageusement, l'acide de formule (II) est un acide gras polyinsaturé, c'est-à-dire comprenant de 1 à 6 insaturations. De manière encore plus avantageuse, il s'agit d'un acide oméga-3.
Parmi ces acides oméga-3 on peut notamment citer l'acide a-linolénique, l'acide cervonique, l'acide timnodonique et l'acide pinolénique. Les acides cervonique, timnodonique et pinolénique sont également connus sous les dénominations respectives d'acide 4,7,10,13,16,19-docosahexaenoïque (DHA), d'acide 5,8,11,14,17-eicosapentaénoïque (EPA) et d'acide 5,9,12- octodécatriénoïque.
Lorsque A représente un radical d'acide monocarboxylique de formule générale (II), il peut être avantageusement choisi parmi l'acide acétique, l'acide myristique, l'acide palmitique, les acides hydroxydécénoïques et décénoïques et notamment, l'acide trans-10-hydroxy-Δ2-décénoïque et l'acide trans-oxo-9-decene-2-oïque.
Avantageusement, l'acide de formule (II) est un acide choisi parmi l'acide lipoïque (Lip) ou sa forme réduite l'acide dihydrolipoïque, la N-lipoyl-lysine ou l'acide phénylbutyrique (Pbu).

De façon avantageuse, A représente le radical correspondant à l'acide palmitique (Palm).

Parmi les conjugués dipeptidiques de l'invention on peut citer les conjugués dipeptidiques choisis dans le groupe constitué par
a) A-Arg-His-NH₂,
b) A-Arg-Arg-NH₂,
c) A-Arg-Pro-NH₂.
d) A-Arg-Lys-NH₂,
e) A-Ser-Pro-NH₂.
f) A-DPhe-Arg-NH₂.
dans lesquelles A à la définition indiquée ci-dessus.

Parmi les conjugués dipeptidiques de l'invention on peut citer les conjugués dipeptidiques choisis dans le groupe constitué par
39) Palm-Arg-His-NH₂,
41) Palm-Arg-Arg-NH₂,
49) Palm-Arg-Pro-NH₂,
50) Palm-Arg-Lys-NH₂,
125) Palm-Ser-Pro-NH₂.
269) Palm-DPhe-Arg-NH₂,
362) Pbu-DPhe-Arg-NH₂,
363) Lip-DPhe-Arg-NH₂.

Les conjugués dipeptidiques objet de la présente invention, peuvent être obtenus soit avantageusement par synthèse chimique classique, soit par synthèse enzymatique, selon des procédés quelconques connus de l'homme du métier.

La présente invention concerne en outre une composition cosmétique, dermatologique ou pharmaceutique ou un complément alimentaire comprenant un conjugué dipeptidique selon la présente invention et éventuellement un excipient cosmétiquement ou pharmaceutiquement acceptable.

Les conjugués dipeptidiques peuvent être administrés pour leur utilisation cosmétique ou pharmaceutique par voie topique. Ils peuvent aussi être utilisés dans des compléments alimentaires, autrement dit dans le domaine nutraceutique par voie orale.

Les conjugués dipeptidiques selon l'invention sont préférentiellement administrés, par voie topique.

La composition cosmétique, pharmaceutique ou dermatologique selon la présente invention destinée à une administration par voie topique pourra se présenter sous les formes qui sont habituellement connues pour ce type d'administration, c'est à dire notamment les lotions, les mousses, les gels, les dispersions, les sprays, les sérums, les masques, les laits corporels, les pommades, les solutions, les émulsions, les gels, ou les crèmes par exemple, avec des excipients permettant notamment une pénétration cutanée afin d'améliorer les propriétés et l'accessibilité du principe actif. Ces compositions contiennent généralement outre le conjugué dipeptidique selon la présente invention, un milieu physiologiquement acceptable, en général à base d'eau ou de solvant, par exemple des alcools, des éthers ou des glycols. Elles peuvent également contenir des agents tensioactifs, des conservateurs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants ou des eaux thermales.

Dans la composition selon la présente invention, le conjugué dipeptidique selon l'invention peut être présent à une concentration comprise entre 10⁻⁸ M et 10⁻³ M, avantageusement comprise entre 10⁻⁷ M et 10⁻⁵ M.

La présente invention concerne de plus un conjugué dipeptidique selon la présente invention ou une composition pharmaceutique selon la présente invention pour son utilisation en tant que médicament, avantageusement destiné à prévenir, améliorer ou traiter les anormalités immunitaires, l'immunodéficience, réguler le poids du corps en contrôlant l'appétit, traiter les désordres du système nerveux central, réguler la satiété, traiter l'anorexie ou certains cancers cutanés.

La présente invention concerne en outre l'utilisation d'une composition cosmétique selon la présente invention en tant que dépigmentant, pour éclaircir ou blanchir l'épiderme, éliminer les tâches de la peau, en particulier de vieillesse ou de rousseur, ou prévenir la pigmentation de l'épiderme.

Enfin, la présente invention concerne un procédé de traitement cosmétique pour éclaircir, dépigmenter ou blanchir l'épiderme, éliminer les tâches de la peau, en particulier de vieillesse ou de rousseur, ou prévenir la pigmentation de l'épiderme comprenant l'application sur la peau d'une composition cosmétique selon la présente invention.

Les exemples suivants sont donnés à titre indicatif non limitatif.

### Exemple 1 : Préparation de 361 dipeptides selon l'invention

Une banque de dipeptides acylés de 361 membres a été synthétisée en utilisant des Lanternes SynPhase™ et une stratégie "split and pool" (séparation et groupage) de marquage de couleur tel que décrit dans l'article de Feliu et al. (J. Comb. Chem., 2003, 5, pages 256-361). Ainsi, ces 361 composés ont été synthétisés sur des Lanternes SynPhase™ de série D avec de la résine Rink amide PS en utilisant la stratégie standard de synthèse Fmoc (9-fluorényl-méthoxycarbonyle) en phase solide utilisant un format d'arrangement Multipin 96. Les blocs constitutifs AA1 et AA2 ont été choisis dans un ensemble chimique de 19 acides aminés D et L comprenant plusieurs types de chaînes latérales (alkyle, aromatiques, acides, volumineuses, basiques) pour produire 19 x 19 = 361 combinaisons.
Les produits chimiques utilisés sont les suivants :
Les acides aminés protégés à l'extrémité N-terminale par un groupe α-Fmoc, Fmoc-Ala-OH, Fmoc-D-Ala-OH, Fmoc-Arg(Pbf)-OH, Fmoc-D-Arg(Pbf)-OH, Fmoc-His(Trt)-OH, Fmoc-D-His(Trt)-OH, Fmoc-Lys(Boc)-OH, Fmoc-Phe-OH, Fmoc-D-Phe-OH, Fmoc-Trp(Boc)-OH, Fmoc-D-Trp(Boc)-OH, Fmoc-Met-OH, Fmoc-Glu(OtBu)-OH, Fmoc-Ser(tBu)-OH, Fmoc-Leu-OH, Fmoc-Tyr(tBu)-OH, Fmoc-Gly-OH, Fmoc-Pro-OH, Fmoc-Asn(trt)-OH, ont été achetés chez SENN chemicals et chez Advanced Chemtech.
L'agent de couplage, le HBTU (hexafluorophosphate de 2-(1-H-benzotriazol-1-yl)-1,1,3,3-tétraméthyl-uronium), a été acheté chez SENN chemicals.
Le N,N-diméthylformamide (DMF), le dichlorométhane, le méthanol, l'acétonitrile, l'éther éthylique, l'acide trifluoroacétique (TFA), la pipéridine ont été achetés chez Riedel-de Haën, Carlo Erba ou Acros organics et utilisés sans purification.
La N,N-diisopropyléthylamine (DIEA), le triisopropylsilane, l'acide palmitique ont été achetés chez Aldrich ou Avocado. Tous les réactifs et produits chimiques étaient de qualité analytique et ils ont été utilisés sans autre purification.
Les Lanternes Polystyrene Rink amide Synphase de la série D ont été fournies par Mimotopes, Pty.

Le procédé standard de fabrication de ces dipeptides comportent les étapes suivantes :

### 1- Protocole standard de déprotection Fmoc

Les étapes de la déprotection Fmoc ont été réalisées en immergeant les lanternes immobilisées sur un support de 96 tiges dans un mélange de diméthylsulfoxide(DMF)/pipéridine (80/20, v/v) pendant 30 minutes. Des récipients rectangulaires en polypropylène de la même taille qu'une plaque standard à 96 puits, ont été utilisé. L'excès de solution de déprotection a été simplement éliminé en agitant vigoureusement le support des tiges.

### 2- Protocole standard de lavage

Après l'étape de couplage ou de déprotection, des étapes de lavage ont été réalisées en plongeant successivement les lanternes arrangées selon un format Multipin dans des récipients en polypropylène contenant du DMF (3 x 5 min), du méthanol (2 x 5 min) et du dichlorométhane (DCM) (1 x 5 min), successivement. Les lanternes ont été séchées à l'air pendant 5 min sous une hotte à vapeurs après le dernier lavage avec du DCM.

### 3- Protocole standard de couplage

Des solutions 0,4 M de chaque acide aminé Fmoc, de HBTU et de DIEA ont été préparées dans du DMF et maintenues à 4°C pendant toute la synthèse. 200 µl de solution d'acide aminé ont été distribués dans des plaques à 96 puits profonds. 200 µl de solution de DIEA et 200 µl de solution de HBTU ont été ajoutés ensuite et finalement, le support des tiges portant les lanternes a été adapté à la plaque à puits profonds pendant 2 heures.

### 4- Clivage

500 µl de solution de TFA/eau/triisopropylsilane (95/2,5/2,5, v/v/v) ont été distribués dans des tubes individuels en polypropylène arrangés dans les plaques Micronic à 96 puits. Le clivage a été réalisé pendant 3 heures. Le cocktail de clivage a été concentré directement à partir des plaques en utilisant une centrifugeuse sous vide Jouan RC1010. Les composés ont été précipités avec de l'éther diéthylique sec, centrifugés et décantés un par un. Les opérations de précipitation, de centrifugation et de décantation ont été répétées deux fois. 500 µl d'acétonitrile/eau (50/50, v/v) contenant 0,1 % de TFA ont été distribués dans chaque tube pour solubiliser les échantillons. Les échantillons ont été ensuite congelés à -80°C et lyophilisés. Cette opération a été répétée deux fois pour éliminer complètement le groupe épurateur triisopropylsilane.

### 5-Préparation et analyse des échantillons

Les banques complètes et les peptides simples resynthétisés ont été analysés par CLHP en phase inverse et CL/SM. 500 µl d'acétonitrile/eau (50/50, v/v) contenant 0,1 % de TFA ont été distribués sur les composés lyophilisés. 10 µl de chaque tube ont été prélevés pour l'analyse de CLHP et de CL/SM ESI+.
Les analyses de CLHP ont été réalisées sur un système de CLHP Waters Alliance 2690 et un détecteur à barrette de photodiodes Waters 996 et une colonne C18 Merck Chromolith Speed ROD de 50 x 4,6 mm. Un débit de 5 ml/min et un gradient de 100 % de B à 100 % de C sur 3 min ont été utilisés (Eluant B, eau/0,1 % de TFA ; Eluant C, acétonitrile/0,1 % de TFA). Les estimations de la pureté sont basées sur le pourcentage de surface des pics détectés à 214 nm.
Le système de CL/SM était constitué d'une CLHP Waters Alliance 2690 couplée à un spectromètre Micromass Platform II (mode ionisation par électronébulisation, ESI+). Toutes les analyses ont été réalisées en utilisant une colonne Waters Symmetry C18, 3,5 µm, 2,1 x 30 mm. Un débit de 600 µl/min et un gradient de 100 % de B à 100 % de C sur 3 min ont été utilisés (Eluant B, eau/0,1 % de TFA ; Eluant C, acétonitrile/0,1 % de TFA).
Les spectres de masse par électronébulisation ionique positive ont été acquis à un débit de solvant de 100 ml/min. De l'azote a été utilisé à la fois pour le gaz nébulisant et pour le gaz séchant. Les données ont été acquises en mode lecture de m/z 400 à 1400 dans des intervalles de 0,1^{-s}; 10 lectures ont été additionnées pour produire le spectre final.
Les poids moléculaires de tous les composés ont été calculés en utilisant des masses mono-isotopiques (C = 12,000, H = 1,007, N = 14,003, O = 15,994, S = 31, 972) .

Les résultats des analyses sont présentés dans le Tableau 1 suivant.

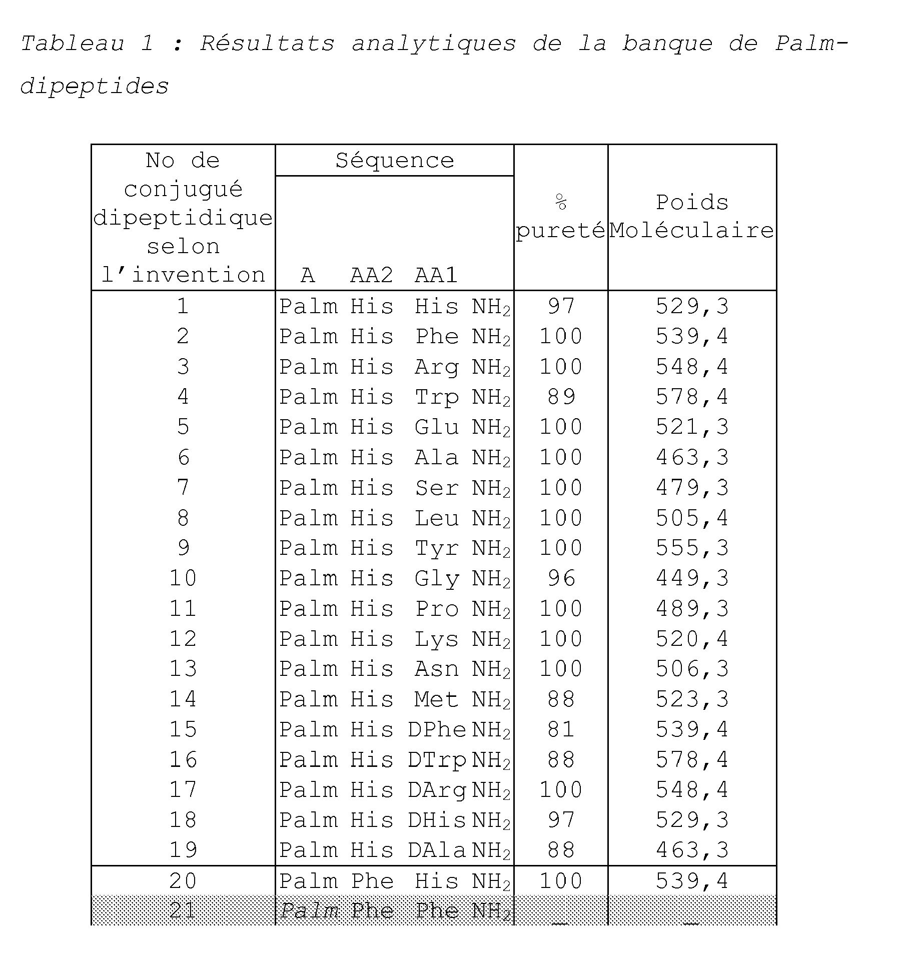

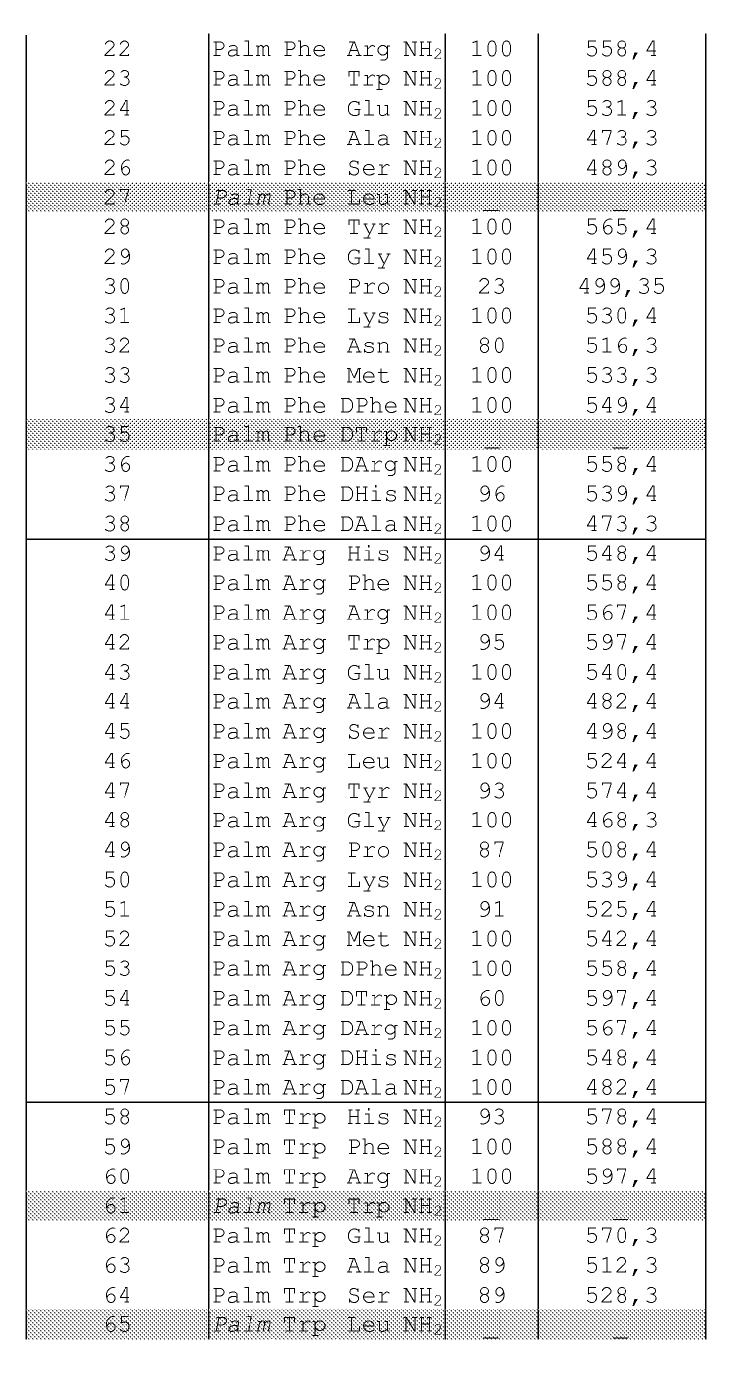

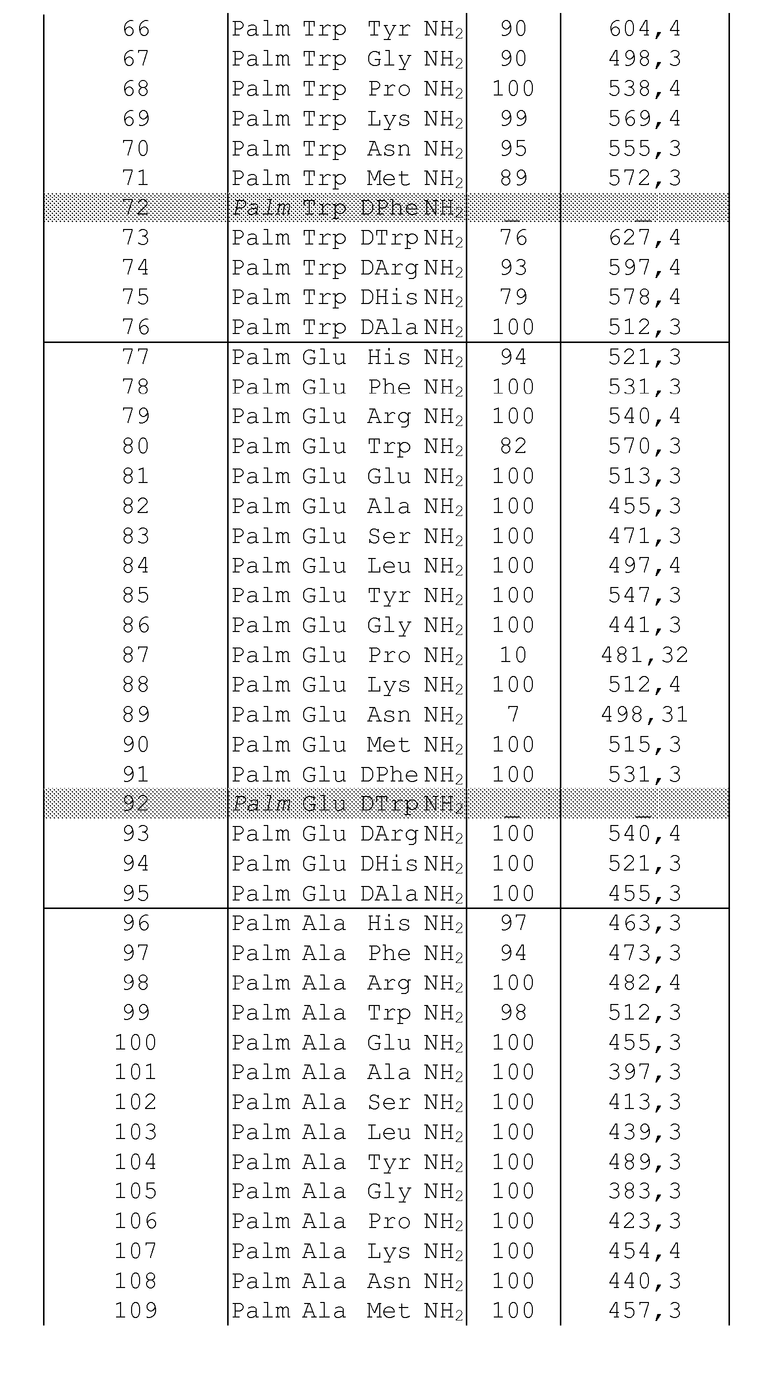

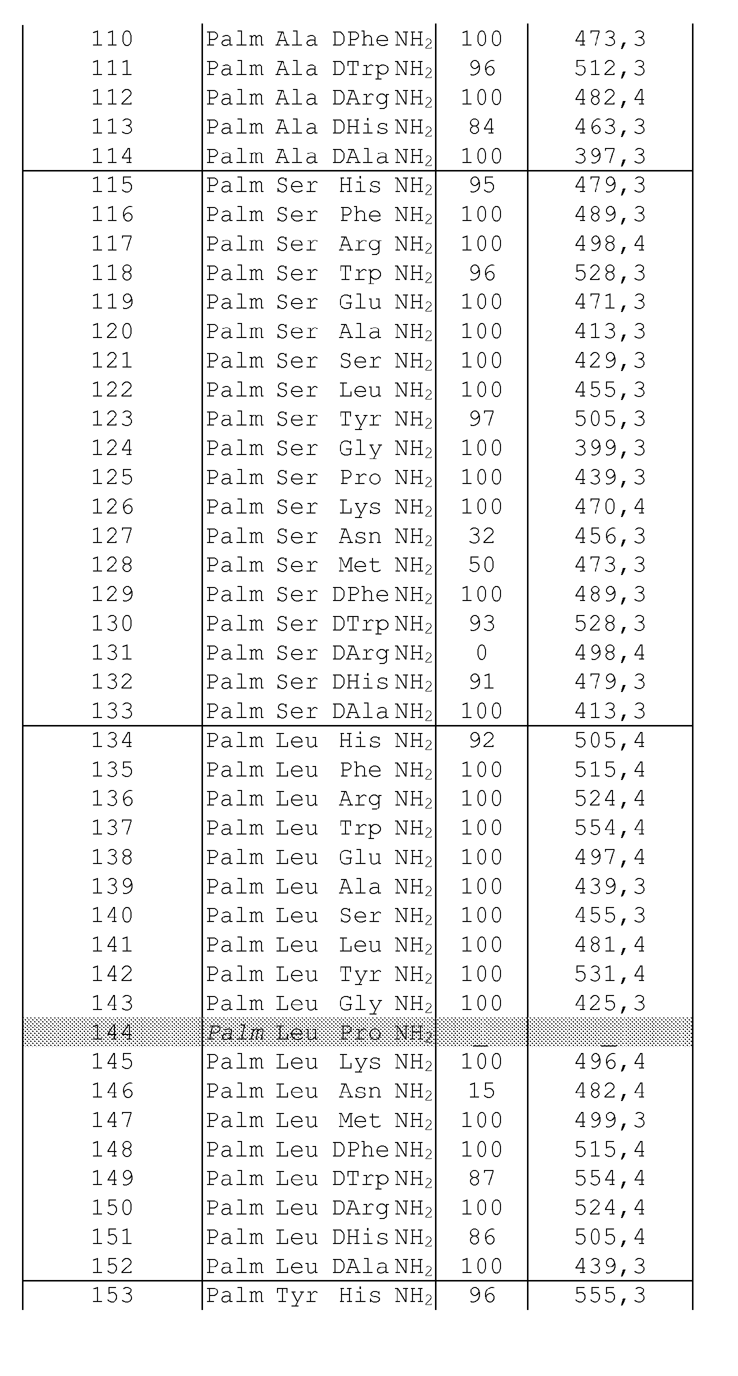

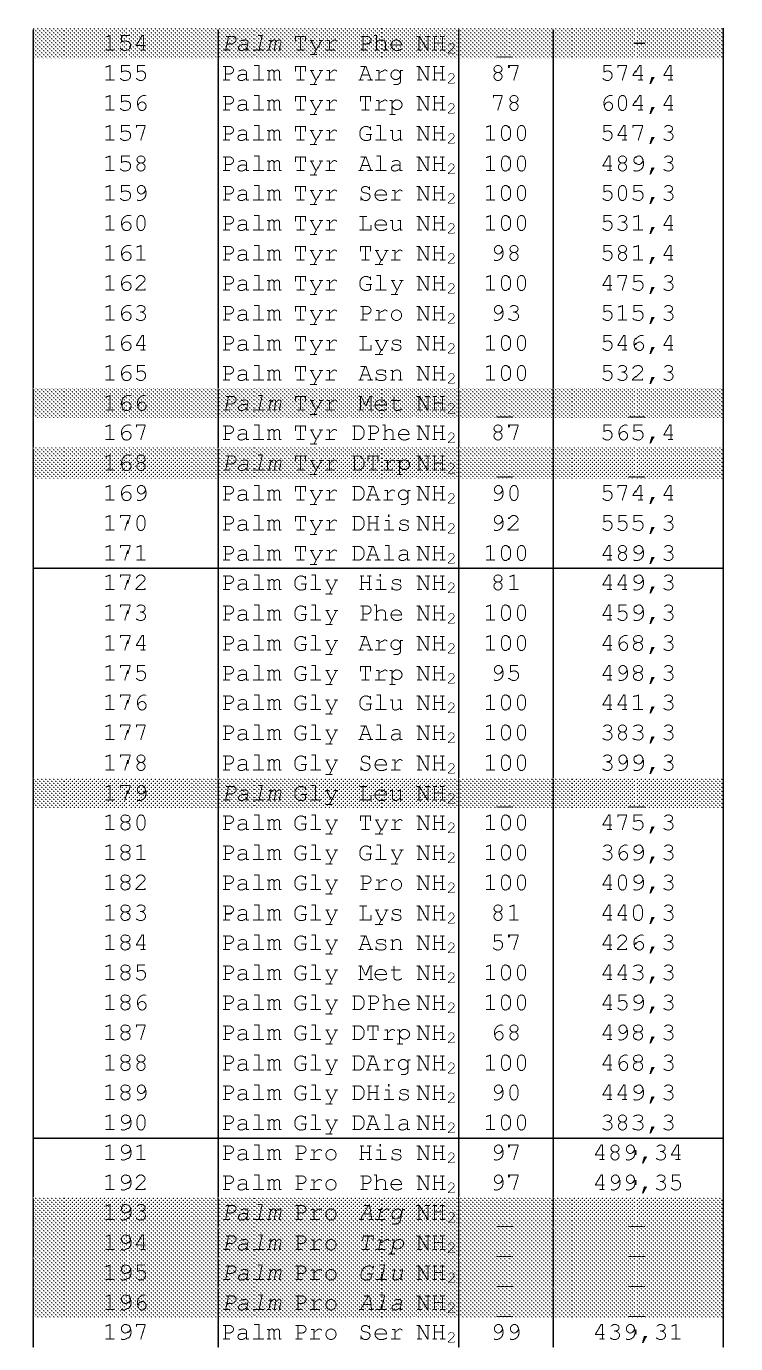

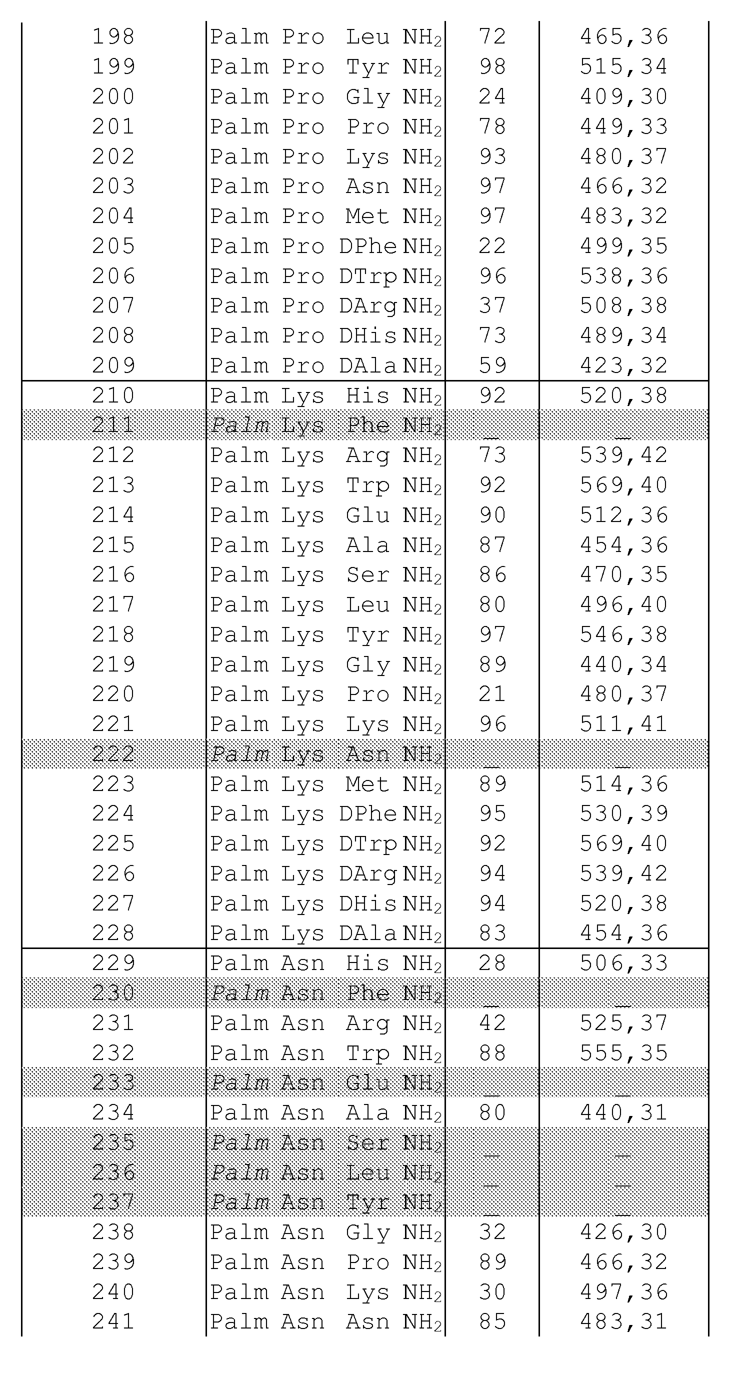

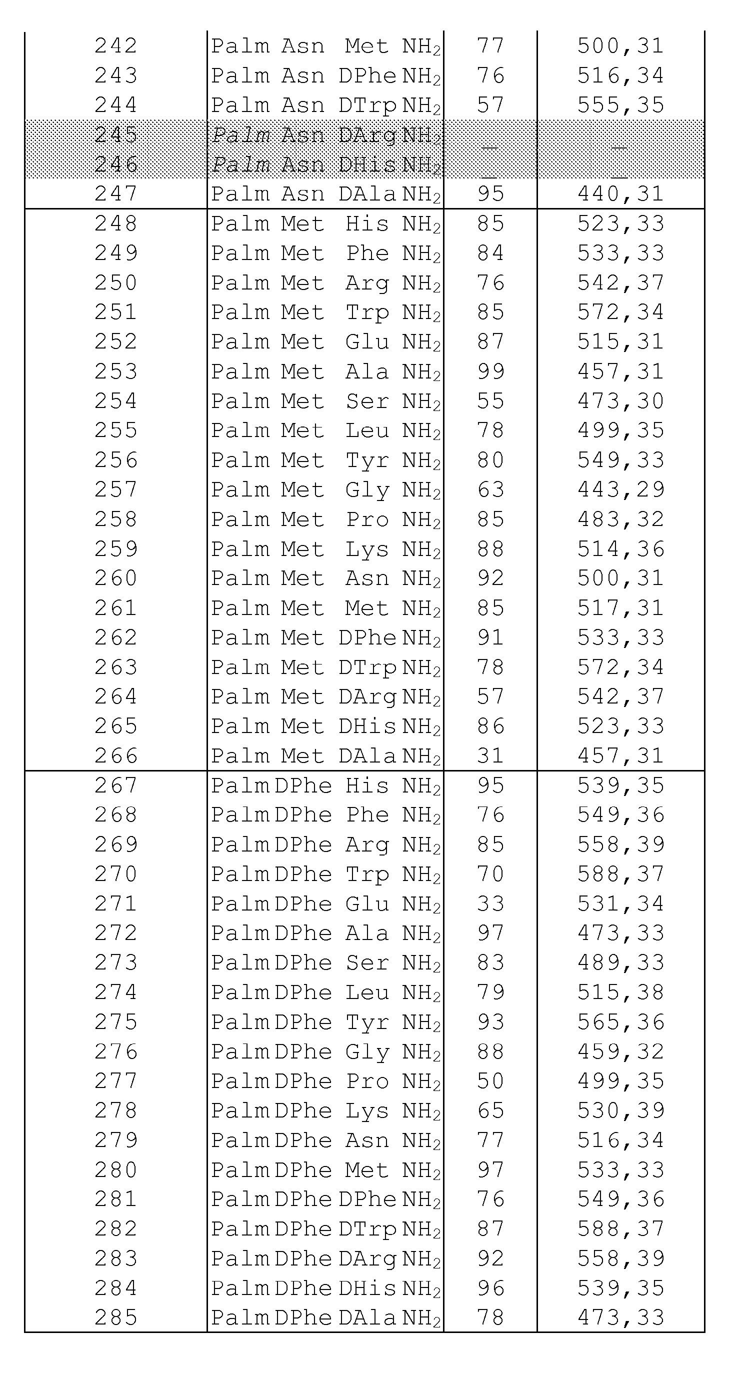

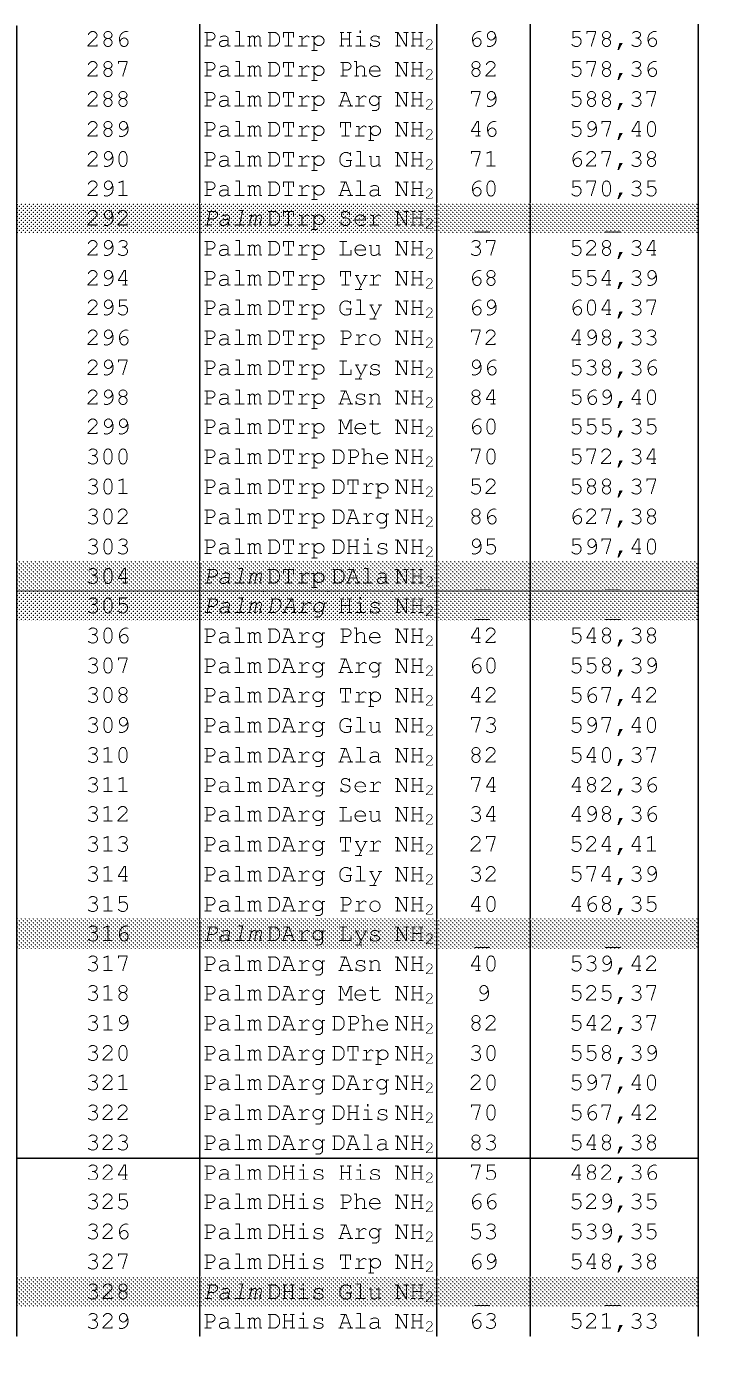

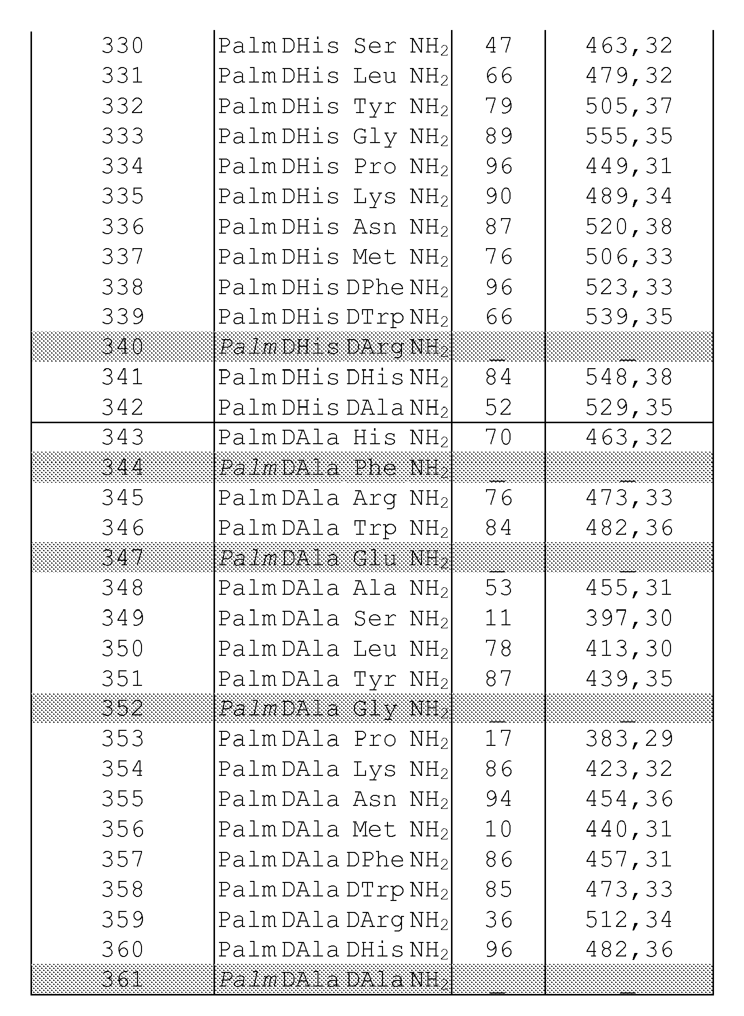

Sauf pour 35 membres de la banque (grisés), tous les composés ont été détectés par analyse ES+ CL/SM. La pureté moyenne déterminée sur la base du pourcentage de la surface du pic attendu détecté à 214 nm sur le chromatogramme est supérieure à 83 %.

### Exemple 2 : Propriétés biologiques de 5 conjugués dipeptides selon la présente invention

Des expériences d'inhibition de la production d'AMPc ont été réalisées sur la lignée cellulaire humaine M4Be avec une concentration de 5.10⁻⁸ M d'α-MSH. 5 Palm-dipeptides selon la présente invention ont été introduits à diverses concentrations et chaque mesure a été faite en triple. Deux ou trois séries d'expériences ont été réalisées.
Les essais ont été réalisés de la façon suivante :
La lignée cellulaire humaine M4Be (Jacubovichet al. Cancer Immunol. Immunother. 1979, 7, 59-64), une lignée cellulaire de mélanocytes capable de produire des mélanines, a été utilisée dans cette étude pour déterminer les valeurs de CI₅₀.
Les cellules ont été maintenues dans du milieu Eagle modifié de Dulbecco avec 10 % de sérum de veau foetal (FCS), 1 mM de glutamine, 100 U/ml de pénicilline et 10⁻⁴ g/ml de streptomycine.
Toutes les lignées cellulaires ont été maintenues à 37°C dans une atmosphère à 5 % de CO₂ et les milieux de culture des cellules ont été renouvelés tous les deux jours. Les cellules ont été plaquées sur une plaque à 96 puits (Nunc, Roskilde) 24 heures avant le contact des dipeptides selon l'invention.
L'AMPc a été mesuré de la façon suivante :
   Les cellules plaquées la veille à 8.10⁴ cellules par puits sont mises en présence d'un des 5 conjugués dipeptidique selon l'invention à diverses concentrations pendant 10 minutes à 37°C avec 5.10⁻⁸ M d'α-MSH. Après ce temps, la lyse des cellules a été réalisée et la teneur en AMPc a été mesurée en utilisant un coffret de test de liaison par compétition (RPN225, Amersham Pharmacia Biotech). Chaque expérience indépendante a été réalisée au moins deux fois en triple.
   L'activité peptidique a été déterminée en se reportant au taux d'AMPc synthétisé par des cellules non traitées et à la production d'AMPc induite par l'α-MSH seule. Les courbes ont été ajustées et les valeurs de CI₅₀ ont été déterminées avec la régression non linéaire dans le GraphPad Prism (logiciel GraphPad, San Diego, CA, Etats-Unis).

Les résultats sont présentés dans le Tableau 2 suivant.

**Tableau 2 : Expériences d'inhibition de la production d'AMPc sur des cellules M4Be**

| **Composés** | **Séquence** | **Pureté (**%**)** | **CI₅₀ (**µ**m) exp1** | **CI₅₀ (**µ**m) exp2** | **CI₅₀ (**µ**m) exp3** | **CI₅₀ (**µ**M) moyenne** |
|---|---|---|---|---|---|---|
| 39 | Palm-Arg-His-NH₂ | 94 | 29 | 4,6 | 4,4 | 13 |
| 41 | Palm-Arg-Arg-NH₂ | 100 | 36 | 17 | - | 26 |
| 49 | Palm-Arg-Pro-NH₂ | 87 | 20 | 26 | - | 23 |
| 50 | Palm-Arg-Lys-NH₂ | 100 | 48 | 9,6 | - | 29 |
| 125 | Palm-Ser-Pro-NH₂ | 100 | 45 | 5,5 | 1,0 | 17 |

Ainsi, de manière inattendue, les Palm-dipeptides testés sont apparus être des antagonistes du récepteur MC₁ humain de la mélanocortine en utilisant des lignées cellulaires de mélanome M4Be. Ces conjugués dipeptidiques présentent une CI₅₀ dans une plage micromolaire. Ces dipeptides sont le premier exemple de composés antagonistes de synthèse courts se liant au récepteur MC₁ et ils ouvrent le champ des antagonistes à petite molécule non peptidique de l'α-MSH.
En particulier ces composés comprennent un résidu arginine en position AA2. De manière intéressante, le composé 125 (Palm-Ser-Pro-NH₂), n'ayant aucun résidu basique dans sa séquence, a montré une valeur de CI₅₀ de 17 µM. Ce résultat a montré que la liaison sur le récepteur MC₁ ne nécessite pas obligatoirement un radical chargé positivement.
Avec seulement deux résidus d'acides aminés, ces composés palmitoylés peuvent être considérés comme des composés chefs de file utiles pour la conception d'antagonistes non peptidiques de l'α-MSH.

## Revendications

1. Composition cosmétique ou pharmaceutique destinée à une administration par voie orale ou par voie topique comprenant un conjugué dipeptidique de formule générale I suivante :
A-AA2-AA1-NH₂ I
dans laquelle
A représente le radical correspondant à un acide monocarboxylique de formule générale II suivante :
HOOC-R II
dans laquelle R représente
- un radical aliphatique en C₁-C₂₄ linéaire ou ramifié, éventuellement substitué par un groupe hydroxyle, pouvant comporter une ou plusieurs insaturations, avantageusement de 1 à 6 insaturations, et/ou pouvant comporter un groupe phényle
- ou l'acide lipoïque ou sa forme réduite l'acide dihydrolipoïque ou la N-lipoyl-lysine,
AA1 et AA2 représentent des acides aminés identiques ou différents choisis dans le groupe constitué par Ala, Asn, Cys, Gln, Gly, Ile, Leu, Met, Phe, Pro, Ser, Thr, Trp, Tyr, Val, Asp, Glu, His, Lys, Orn, Dap, Dab les homoacides aminés correspondants et les béta-acides aminés correspondants
sous forme d'énantiomères ou de diastéréoisomères ainsi que leurs mélanges, y compris les mélanges racémiques, à la condition qu'au moins un des acides aminés AA1 ou AA2 est Lys
et un excipient cosmétiquement ou pharmaceutiquement acceptable.

2. Composition cosmétique ou pharmaceutique selon la revendication 1 **caractérisée en ce que** AA2 représente un acide aminé choisi dans le groupe constitué par His, Lys, Orn, Dap ou Dab.

3. Composition cosmétique ou pharmaceutique selon la revendication 1 ou 2 **caractérisée en ce que** A représente le radical correspondant à l'acide palmitique.

4. Composition cosmétique ou pharmaceutique selon l'une quelconque des revendications 1 à 3 **caractérisée en ce que** le conjugué dipeptidique de formule générale I est choisi dans le groupe constitué par
12) Palm-His-Lys-NH₂,
31) Palm-Phe-Lys-NH₂,
69) Palm-Trp-Lys-NH₂,
88) Palm-Glu-Lys-NH₂,
107) Palm-Ala-Lys-NH₂,
126) Palm-Ser-Lys-NH₂,
145) Palm-Leu-Lys-NH₂,
164) Palm-Tyr-Lys-NH₂,
183) Palm-Gly-Lys-NH₂,
202) Palm-Pro-Lys-NH₂,
210) Palm-Lys-His-NH₂,
213) Palm-Lys-Trp-NH₂,
214) Palm-Lys-Glu-NH₂,
215) Palm-Lys-Ala-NH₂,
216) Palm-Lys-Ser-NH₂,
217) Palm-Lys-Leu-NH₂,
218) Palm-Lys-Tyr-NH₂,
219) Palm-Lys-Gly-NH₂,
220) Palm-Lys-Pro-NH₂,
221) Palm-Lys-Lys-NH₂,
223) Palm-Lys-Met-NH₂,
224) Palm-Lys-DPhe-NH₂,
225) Palm-Lys-DTrp-NH₂,
226) Palm-Lys-DArg-NH₂,
227) Palm-Lys-DHis-NH₂,
228) Palm-Lys-DAla-NH₂,
240) Palm-Asn-Lys-NH₂,
259) Palm-Met-Lys-NH₂,
278) Palm-DPhe-Lys-NH₂,
297) Palm-DTrp-Lys-NH₂,
335) Palm-DHis-Lys-NH₂,
354) Palm-DAla-Lys-NH₂.

5. Composition pharmaceutique selon l'une quelconque des revendications 1 à 4 à titre de médicament, avantageusement destinée à prévenir, améliorer ou traiter les anormalités immunitaires, l'immunodéficience, réguler le poids du corps en contrôlant l'appétit, traiter les désordres du système nerveux central, réguler la satiété, traiter l'anorexie ou traiter certains cancers cutanés.

6. Utilisation d'une composition cosmétique selon l'une quelconque des revendications 1 à 4 en tant que dépigmentant, pour éclaircir ou blanchir l'épiderme, éliminer les tâches de la peau, en particulier de vieillesse ou de rousseur, ou prévenir la pigmentation de l'épiderme.

7. Procédé de traitement cosmétique pour éclaircir, dépigmenter ou blanchir l'épiderme, éliminer les tâches de la peau, en particulier de vieillesse ou de rousseur, ou prévenir la pigmentation de l'épiderme comprenant l'application sur la peau d'une composition cosmétique selon l'une quelconque des revendications 1 à 4.
